(19) Europäisches Patentamt
European Patent Office
Office européen des brevets

(11) **EP 2 103 939 A1**

(12) **EUROPEAN PATENT APPLICATION**

(43) Date of publication:
**23.09.2009 Bulletin 2009/39**

(51) Int Cl.:
***G01N 33/58*** *(2006.01)* ***G01N 33/68*** *(2006.01)*

(21) Application number: **08290265.1**

(22) Date of filing: **20.03.2008**

(84) Designated Contracting States:
**AT BE BG CH CY CZ DE DK EE ES FI FR GB GR HR HU IE IS IT LI LT LU LV MC MT NL NO PL PT RO SE SI SK TR**
Designated Extension States:
**AL BA MK RS**

(71) Applicant: **sanofi-aventis**
**75013 Paris (FR)**

(72) Inventor: **The designation of the inventor has not yet been filed**

(74) Representative: **Schneider, Rudolf et al**
**Sanofi-Aventis Deutschland GmbH**
**Patente Deutschland**
**Industriepark Höchst**
**Gebäude K 801**
**D-65926 Frankfurt am Main (DE)**

(54) **Fluorescence based assay to detect sodium/calcium exchanger (NCX) "reverse mode" modulating compounds**

(57) Transporters are an emerging target family with enormous potential, offering scientific and economic opportunities. The sodium/calcium exchanger is an important mechanism for removing $Ca^{2+}$ from diverse cells. In heart, it extrudes $Ca^{2+}$ that has entered through $Ca^{2+}$ channels to initiate contraction, while $Na^+$ enters the heart cell. It is of considerable interest to identify compounds that modulate, not only the calcium export activity (forward mode), but also the calcium import activity (reverse mode) of sodium/calcium exchangers.

The present invention is directed to a fluorescence-based assay for detecting NCX "reverse mode" modulating compounds. It further refers to a kit of parts comprising cells expriming NCX and the use of the kit of parts to test a compound for activity as an agonist or antagonist of NCX.

EP 2 103 939 A1

**Description**

FIELD OF THE INVENTION

The present invention relates to sodium/calcium exchangers (NCX) and methods for determining their activity. More specifically, the invention relates to a fluorescence-based assay for detecting NCX "reverse mode" modulating compounds. It further refers to a kit of parts comprising cells expriming NCX and the use of the kit of parts.

BACKGROUND OF THE INVENTION

A basic requirement for life is compartmentalization - with biological membranes being nature's tool to realize this principle. However, a lipid bilayer - the structure underlying the cell membrane - is impermeable to most ions and compounds whose transport is essential to sustain vital functions in cells and organisms. The answer to this paradox lies in the semipermeable nature of the cell membrane - solutes that have to cross the membrane are transported by specific membrane proteins. These transporters are responsible for the generation and maintenance of ion gradients, the uptake of nutrients, the transport of metabolites, the reuptake of signaling molecules and the disposal of toxic and waste compounds. Therefore, transporters are potential drug targets that allow direct influence on disease-related abnormalities in this context.

Transporters are an emerging target family with enormous potential, offering scientific and economic opportunities. On the other hand, transporters are a difficult target class in terms of drug-discovery technologies.

The sodium/calcium exchanger human gene family (also known as NCX or SLC8) encompasses three distinct proteins, NCX1, NCX2 and NCX3. SLC8 together with SLC24 constitute a superfamily of $Na^+/Ca^{2+}$ countertransporters. SLC24 family members also transport $K^+$, they are also known as NCKXs.

NCX1 is the most highly characterized member of the sodium/calcium exchanger human gene family, its expression is up regulated in failing human heart and is involved in ischemia-reperfusion damage after myocardial infarction. Inhibition of NCX1 normalizes heart muscle contractility in failing hearts and acts cardio-protective during post-ischemic reperfusion (Flesch et al., Circulation 1996; Komuro and Ohtsuka, Journal of Pharmacological. Sciences. 2004). NCX2 is mainly expressed in the brain and NCX3 in the brain and skeletal muscle, their physiological roles remain elusive.

The sodium/calcium exchanger can transport $Ca^{2+}$ and $Na^+$ in two directions depending on membrane potential and ion gradients. At the first direction, named as "forward mode" or "calcium export mode", $Ca^{2+}$ is transported out of the cell and $Na^+$ into the cell. At the other direction, named "reverse mode" or "calcium import mode", the transport directions are vice versa.

The sodium/calcium exchanger is an important mechanism for removing $Ca^{2+}$ from diverse cells. In heart, it extrudes $Ca^{2+}$ that has entered through $Ca^{2+}$ channels to initiate contraction, while $Na^+$ enters the heart cell. Its relevance in cardiovascular diseases is e.g. illustrated in Hobai, JA & O'Rourke,B (2004) Expert Opin. Investig. Drugs, 13, 653-664. Therefore, pharmaceutical industry has developed compounds inhibiting the NCX as e.g. described in Iwamoto, T. et al. (2004) J. Biol. Chem., 279, 7544-7553.

The inhibition of the cardiac sodium/calcium exchanger is important for cardio-protection (e.g. Pogwizd, 2003). It exists a medical need to discrimate between compounds inhibiting the "forward mode" and compounds inhibiting the "reverse mode" of the NCXs. Thus, an assay analyzing the calcium import mode is needed. In addition, compounds inhibiting NCX1 should be selective as NCX2 and NCX3 belong to the same transporter family. Thus, an assay allowing to do selectivity profiling is needed.

It is of considerable interest to identify compounds that modulate the sodium/calcium exchanger activity, for example, by blocking the flow of calcium and/or inhibiting the activation of NCX. One standard method to do so is through the use of patch clamp experiments.

In these experiments, cells must be evaluated individually and in sequence by highly skilled operators, by measuring the calcium current across the cell membrane in response to changes of the membrane potential and/or application of test compounds. The effect of Sea0400, a new specific inhibitor of NCX, on the action potential in dog ventricular papillary muscle was investigated and disclosed by K. Acsai during the "ESC Congress 2004" in Munich on Poster Nr. 2886 (Title: Effect of a specific sodium-calcium exchanger blocker Sea0400 on the ventricular action potential and triggered activity in dog ventricular muscle and Purkinje fiber) and by C. Lee et al. (The journal of pharmacology and experimental therapeutics; Vol. 311: 748-757, 2004; Title: Inhibitory profile of SEA0400 [2-[4-[(2,5-Difluorophenyl)methoxy]phenoxy]-5-ethoxyaniline] assessed on the cardiac $Na^+/Ca^{2+}$ exchanger, NCX1.1).

It was shown, using an ion-selective electrode technique to quantify ion fluxes in giant patches, that the cardiac $Na^+/Ca^{2+}$ exchanger has multiple transport modes (Tong Mook Kang & Donald W. Hilgemann; Nature; Vol. 427, 5 February 2004; Title: Multiple transport modes of the cardiac $Na^+/Ca^{2+}$ exchanger).

These experiments, while valid and informative, are very time consuming and not adaptable to high-throughput assays for compounds that modulate calcium ion channel activity.

Various techniques have been developed as alternatives to standard methods of electrophysiology. For example, radioactive flux assays have been used in which cells are exposed with a radioactive tracer (e.g., $^{45}$Ca) and the flux of the radio-labled Ca is monitored. Cells loaded with the tracer are exposed to compounds and those compounds that either enhance or diminish the efflux of the tracer are identified as possible activators or inhibitors of ion channels in the cells' membranes. A specific example is enclosed in T. Kuramochi et al.; Bioorganic & Medicinal Chemistry; 12 (2004) 5039-5056; Title: Synthesis and structure-activity relationships of phenoxypyridine derivates as novel inhibitors of the sodium-calcium exchanger. EP1031556 discloses a method wherein $Na^+/Ca^{2+}$ exchanger activity is measured using sarcolemmal vesicles, the concentration of $Ca^{2+}$ uptake in the sarcolemmal vesicles being determined by measuring $^{45}$Ca radioactivity.

Many radioactive ion-transporter assays have limited sensitivity and therefore insufficient data quality. In addition, the cost and safety issues associated with the radioactive screening technology are hurdles that hinder a broadened application.

Among the above cited drug-discovery technologies, the use of radioactive flux assays to identify compounds that modulate the activity of ion channels and ion transporters is the closest prior art to our invention as it is a technique in which a test compound can be identified as possible activator or inhibitor by monitoring the flux of $Ca^{2+}$ from the cells.

The main issue for the radioactive assays is based on the difficulty of detecting the limited turnover of ion transporters of about 1 to 1000 molecules per second - about $10^4$ times less than most ion channels.

The problem arising from the state of the art was therefore to identify a robust assay for high throughput screening and profiling of sodium/calcium exchanger modulators with a very good sensitivity, allowing to discriminate the "forward mode" and "reverse mode" modulating activity of compounds and allowing to profile identified modulators regarding their selectivity towards NCX1, NCX2 and NCX3, respectively. The present invention solves this problem.

SUMMARY OF THE INVENTION

One subject-matter of the present invention refers to an assay for determining the calcium import activity of a sodium/calcium exchanger wherein:

> a) cells expressing a sodium/calcium exchanger are provided;
> b) a colored substance for determining intracellular calcium is provided;
> c) cells are contacted with a sodium/calcium exchanger activator; and
> d) the calcium mediated change in the luminescent signal from said colored substance is compared to a luminescent signal produced in a control experiment.

Another subject-matter of the present invention refers to an assay for determining the calcium import activity of a sodium/calcium exchanger in response to the addition of a compound wherein:

> a) cells expressing a sodium/calcium exchanger are provided;
> b) a colored substance for determining intracellular calcium is provided;
> c) cells are contacted with a compound, wherein said cells have been treated, prior to treating with said compound, with a sodium/calcium exchanger activator; and
> d) the calcium mediated change in the luminescent signal from said colored substance is compared to a luminescent signal produced in a control experiment.

In general, the sodium/calcium exchanger used is of mammalian origin, and in particular of human origin. The sodium/calcium exchanger is selected from one of the following sodium/calcium exchanger proteins: NCX1, NCX2, NCX3, NCX4, NCX5, NCX6 and/or NCX7, in particular NCX1, NCX2 and/or NCX3; and/or from one of the following sodium/calcium/potassium exchanger proteins: NCKX1, NCKX2, NCKX3, NCKX4 and/or NCKX5.

In general, the cells used in the assay of the present invention can be derived from any eukaryotic organism. In a preferred embodiment, the cells are mammalian cells. In a more preferred embodiment, the cells are CHO (CCL-61), HEK (CCL-1573), COS7 (CRL-1651) and/or JURKAT (CRL-1990) cells.

In a preferred embodiment, said colored substance is added to the cells as a dye precursor capable of entering the cells and being hydrolyzed to a dye, whereby the dye complexes with calcium in said cells and provides a luminescent signal. Further said dye precursor can be preferably an acetoxymethylester derivate and said dye can be preferably the calcium sensitive fluorescence dye fluo-4. In a more preferred embodiment, said luminescent signal is fluorescence and said monitoring step c) employs a FLIPR device.

The invention pertains further to the use of an assay as mentioned before to test a compound for activity as an agonist or antagonist of the calcium import activity of a sodium/calcium exchanger. In another preferred embodiment, the invention pertains to the use of an assay as mentioned before for the diagnosis of a disease associated with a sodium/calcium exchanger altered expression.

The invention pertains further to a kit of parts comprising:

a) lyophilized cells expriming a sodium/calcium exchanger;
b) a colored substance;
c) a compound buffer; and
d) a colored substance buffer.

In a preferred embodiment of the kit of parts of the present invention, said colored substance is the calcium sensitive fluorescence dye fluo-4. In another preferred embodiment, the sodium/calcium exchanger used is of mammalian origin, and in particular of human origin. The sodium/calcium exchanger is selected from one of the following sodium/calcium exchanger proteins: NCX1, NCX2, NCX3, NCX4, NCX5, NCX6 and/or NCX7, in particular NCX1, NCX2 and/or NCX3; and/or from one of the following sodium/calcium/potassium exchanger proteins: NCKX1, NCKX2, NCKX3, NCKX4 and/or NCKX5.

The invention pertains further to the use of a kit of parts as mentioned before to test a compound for activity as an agonist or antagonist of the calcium import activity of a sodium/calcium exchanger. In another preferred embodiment, the invention pertains to the use of a kit of parts as mentioned before for the diagnosis of a disease associated with a sodium/calcium exchanger altered expression.

DETAILED DESCRIPTION OF THE INVENTION

The term "assay" refers to a procedure where a property of a system or object is measured. Assay is a short hand commonly used term for biological assay and is a type of in vitro experiment. Assays are typically conducted to measure the effects of a substance on a living organism. Assays may be qualitative or quantitative, they are essential in the development of new drugs.

The subject assay provides a broad dynamic range so that the activity of a NCX protein can be determined. In particular the present invention makes available a rapid, effective assay for screening and profiling pharmaceutically effective compounds that specifically interact with and modulate the activity of a sodium/calcium exchanger.

The term " sodium/calcium exchanger " or "NCX" in context of the present invention shall mean any one of the list of the following $Na^+/Ca^{2+}$ exchanger proteins: NCX1, NCX2, NCX3, NCX4, NCX5, NCX6, NCX7; or any one of the list of the following $Na^+/Ca^{2+}/K^+$ exchanger proteins: NCKX1, NCKX2, NCKX3, NCKX4, NCKX5, either alone or in combination with each other.

Especially preferred are the SLC8 family members NCX1, NCX2 and/or NCX3 which amino acid sequences correspond, respectively, to SEQ ID NO: 1, SEQ ID NO: 2 and SEQ ID NO: 3.

Such a sodium/calcium exchanger could be derived from any vertebrate and in particular mammalian species (e.g. dog, horse, bovine, mouse, rat, canine, rabbit, chicken, anthropoid, human or others). The sodium/calcium exchanger could be isolated from tissue probes of such vertebrate organisms or could be manufactured by means of recombinant biological material that is able to express the sodium/calcium exchanger.

The term "sodium/calcium exchanger protein" refers to polypeptides, polymorphic variants, mutants, and interspecies homologues that have an amino acid sequence that has greater than about 80% amino acid sequence identity, 85%, 90%, preferably 91%, 92%, 93%, 94%, 95%, 96%, 97%, 98% or 99% or greater amino acid sequence identity, preferably over a region of at least about 25, 50, 100, 200, or 500, or more amino acids, to an amino acid sequence contained in SEQ ID NO: 1, SEQ ID NO: 2 and SEQ ID NO: 3.

The term "biological material" means any material containing genetic information and capable of reproducing itself or being reproduced in a biological system. Recombinant biological material is any biological material that was produced, has been changed or modified by means of recombinant techniques well known to a person skilled in the art.

The following references are examples of the cloning of particular NCX proteins: The canine $Na^+/Ca^{2+}$ exchanger NCX1 has been cloned by Nicoll, DA. et al. (Science. 250(4980): 562-5, 1990; Title: Molecular cloning and functional expression of the cardiac sarcolemmal. Na(+)-Ca2+ exchanger.). The human $Na^+/Ca^{2+}$ exchanger NCX1 has been cloned by Komuro, I., et al. (Proc. Natl. Acad. Sci. U.S.A. 89 (10), 4769- 4773, 1992; Title: Molecular cloning and characterization of the human cardiac $Na^+/Ca^{2+}$ exchanger cDNA) and by Kofuji, P. et al. (Am. J. Physiol. 263 (Cell Physiol. 32): C1241-C1249, 1992; Title: Expression of the Na-Ca exchanger in diverse tissues: a study using the cloned human cardiac Na-Ca exchanger). The human $Na^+/Ca^{2+}$ exchanger NCX2 has been cloned by Li, Z. et al. (J. Biol. Chem. 269(26): 17434-9, 1994; Title: Cloning of the NCX2 isoform of the plasma membrane Na(+)-Ca2+ exchanger).

The rat $Na^+/Ca^{2+}$ exchanger NCX3 has been cloned by Nicoll, DA. et. al. (J. Biol. Chem. 271(40): 24914-21. 1996; Title: Cloning of a third mammalian $Na^+/Ca^{2+}$ exchanger, NCX3). The human $Na^+/Ca^{2+}$ exchanger NCX3 has been cloned by Gabellini, N. et. al. (Gene. 298: 1-7, 2002; Title: The human SLC8A3 gene and the tissue-specific $Na^+/Ca^{2+}$ exchanger 3 isoforms).

The terms "polypeptide", "peptide" and "protein" are used interchangeably herein to refer to a polymer of amino acid residues. The terms apply to amino acid polymers in which one or more amino acid residue is an artificial chemical mimetic of a corresponding naturally occurring amino acid, as well as to naturally occurring amino acid polymers and non-naturally occurring amino acid polymer.

The term "calcium import activity of a sodium/calcium exchanger" refers to the "reverse mode" of the sodium/calcium exchanger, i.e. the mechanism of transporting $Ca^{2+}$ into the cell and $Na^+$ out of the cell. This "reverse mode" transport occurs under certain plasma membrane-depolarizing conditions and high cytosolic $Na^+$ concentration.
The activity of a sodium/calcium exchanger is determined by measuring the enhanced luminescence resulting from a suitable colored substance complexing with calcium.

The term "cells expressing a sodium/calcium exchanger" refers to cells expressing the exchanger of interest endogenously or recombinant cells.

The term "recombinant" when used with reference, e. g., to a cell, or nucleic acid, protein, or vector, indicates that the cell, nucleic acid, protein or vector, has been modified by the introduction of a heterologous nucleic acid or protein or the alteration of a native nucleic acid or protein, or that the cell is derived from a cell so modified. Thus, for example, recombinant cells express genes that are not found within the native (non-recombinant) form of the cell or express native genes that are otherwise abnormally expressed, under expressed or not expressed at all. In the present invention this typically refers to cells that have been transfected with nucleic acid sequences that encode a sodium/calcium exchanger.

The assay is performed simply by growing the cells in an appropriate container with a suitable culture medium. The cell may be a naturally occurring cell, a native cell, an established cell line, a commercially available cell, a genetically modified cell, etc. so long as the cell is able to be maintained during the assay and desirably growing in a culture medium.

Suitable cells for generating the subject assay include prokaryotes, yeast, or higher eukaryotic cells, especially mammalian cells. Prokaryotes include gram negative and gram positive organisms. The cells will usually be mammalian cells, such as human cells, mouse cells, rat cells, Chinese hamster cells, etc. Cells that are found to be convenient include CHO, COS7, JURKAT, HeLa, HEKs, MDCK and HEK293 cells.
Cells may be prepared with the well known methods (Current protocols in cell biology, John Wiley & Sons Inc, ISBN: 0471241059) or may be bought (Invitrogen Corp., Sigma-Aldrich Corp., Stratagene).

The term "colored substance" refers in particular to a calcium sensitive fluorescence dye. The dye precursor is characterized by not being luminescent under the conditions of the assay, being an ester capable of entering the cells and that is hydrolyzed intracellularly to the luminescent oxy compound, and providing enhanced luminescence upon complexing with calcium. The esters are chosen to be susceptible to hydrolysis by intracellular hydrolases.

The term "capable of entering the cells" means that the precursors are able to cross the cellular membrane and be hydrolyzed in the cells, the dye precursor enters the cells under specific conditions of pH, temperature, etc., enters the cells at different speeds or does not enter the cells under specific conditions.

The colored substance is added to the cells using the well known protocols (Current protocols in cell biology, John Wiley & Sons Inc, ISBN: 0471241059).
The use of a colored substance is conventional and commercially available reagents (Invitrogen Corp.) as well as reagents synthesized in laboratory can be used.
A number of commercially available dyes fulfilling the above requirements are known. Fluorescent dyes for monitoring $Ca^{2+}$ are well known and described in detail in section 20.1-20.4 of the Molecular Probes catalog, 9th edition. They usually have two bis-carboxymethylamino groups attached to a fluorescent nucleus such as fluoresceins, rhodamines, coumarins, aminophenylindoles, and others. For the most part the compounds are 3,6-dioxy substituted xanthenes, where in the precursor the oxy groups are substituted and in the luminescent dye they are unsubstituted. Usually there are acetoxymethyl groups protecting the phenols and acids. See, for example, Fluo3/4, Fura2/3, calcein green, etc. Hydrolysis of the acetyl group results in the luminescent product. The precursors are able to cross the cellular membrane and be hydrolyzed in the cell.

The term "luminescence" refers to a "cold light", light from other sources of energy, which can take place at normal and lower temperatures. In luminescence, some energy source kicks an electron of an atom out of its "ground" (lowest-energy) state into an "excited" (higher-energy) state; then the electron gives back the energy in the form of light so it can fall back to its "ground" state. There are several varieties of luminescence, each named according to what the source of energy is, or what the trigger for the luminescence is.

The term "fluorescence" refers to a luminescence that is mostly found as an optical phenomenon in cold bodies, in which the molecular absorption of a photon triggers the emission of another photon with a longer wavelength. The energy difference between the absorbed and emitted photons ends up as molecular vibrations or heat. Usually the absorbed photon is in the ultraviolet range, and the emitted light is in the visible range, but this depends on the absorbance curve and Stokes shift of the particular fluorophore. Fluorescence is named after the mineral fluorite, composed of calcium fluoride, which often exhibits this phenomenon.

Fluorescence from the indicator dyes can be measured with a luminometer or a fluorescence imager. One preferred detection instrument is the Fluorometric Imaging Plate Reader (FLIPR) (Molecular Devices, Sunnyvale, Calif.). The FLIPR is well suited to high throughput screening using the methods of the present invention as it incorporates integrated liquid handling capable of simultaneously pipetting to 96 or 384 wells of a microtiter plate and rapid kinetic detection using a argon laser coupled to a charge-coupled device imaging camera.

An alternative to the use of calcium indicator dyes is the use of the aequorin system. The aequorin system makes use of the protein apoaequorin, which binds to the lipophilic chromophore coelenterazine forming a combination of apoaequorin and coelenterazine that is known as aequorin. Apoaequorin has three calcium binding sites and, upon calcium binding, the apoaequorin portion of aequorin changes its conformation. This change in conformation causes coelenterazine to be oxidized into coelenteramide, $CO_2$, and a photon of blue light (466 nm). This photon can be detected with suitable instrumentation. For reviews on the use of aequorin, see Créton et al., 1999, Microscopy Research and Technique 46:390-397; Brini et al., 1995, J. Biol. Chem. 270:9896-9903; Knight & Knight, 1995, Meth. Cell. Biol. 49: 201-216. Also of interest may be U.S. Pat. No. 5,714,666 which describes methods of measuring intracellular calcium in mammalian cells by the addition of coelenterazine co-factors to mammalian cells that express apoaequorin.

"Inhibitors" are compounds that, e. g., bind to, partially or totally block activity, decrease, prevent, delay activation, inactivate, desensitize, or down regulate the activity or expression of sodium/calcium exchanger proteins, e. g., antagonists.

"Activators" are compounds that increase, open, activate, facilitate, enhance activation, sensitize, agonize, or up regulate sodium/calcium exchanger activity. A preferred activator in the present invention is gramicidine, which triggers the elevation of the intracellular sodium concentration, thus leading to an increase of the sodium/calcium exchanger activity in the "reverse" transport mode.

Inhibitors, activators, or modulators also include genetically modified versions of sodium/calcium exchanger proteins, e. g., versions with altered activity, as well as naturally occurring and synthetic ligands, antagonists, agonists, peptides, cyclic peptides, nucleic acids, antibodies, antisense molecules, ribozymes, small organic molecules and the like.

The term "compound" or "test compound" or "test candidate" or grammatical equivalents thereof describes any molecule, either naturally occurring or synthetic, e. g., protein, oligopeptide, small organic molecule, polysaccharide, lipid, fatty acid, polynucleotide, oligonucleotide, etc., to be tested for the capacity to modulate sodium/calcium exchanger activity (Current protocols in molecular biology, John Wiley & Sons Inc, ISBN: 0471250961). The test compound can be in the form of a library of test compounds, such as a combinatorial or randomized library that provides a sufficient range of diversity (Current protocols in molecular biology, John Wiley & Sons Inc, ISBN: 0471250937). Test compounds are optionally linked to a fusion partner, e. g., targeting compounds, rescue compounds, dimerization compounds, stabilizing compounds, addressable compounds, and other functional moieties. Conventionally, new chemical entities with useful properties are generated by identifying a test compound (called a "lead compound") with some desirable property or activity, e. g., enhancing activity, creating variants of the lead compound, and evaluating the property and activity of those variant compounds. Preferably, high throughput screening (HTS) methods are employed for such an analysis.

Said inhibitor, activator and test compound may be added to the cells by injection into the culture medium after the cells have grown or they may be present in the culture medium prior to the cell growth (Current protocols in cell biology, John Wiley & Sons Inc, ISBN: 0471241059).

The cells may be grown to the appropriate number on the inhibitor, activator and/or test compound or they may be placed on it and used without further growth. The cells may be attached to the inhibitor, activator and/or test compound or, in those embodiments where the cells are placed or grown in wells, the cells may be suspension cells that are suspended in the fluid in the wells.

The term "control experiment" refers to different kinds of experiments that should be run together. The skilled person will recognize that it is generally beneficial to run controls together with the methods described herein.

For example, it will usually be helpful to have a control for the assay for determining the activity of a sodium/calcium exchanger in which the cells are preferably essentially identical to the cells that are used in the assay except that these cells would not express the sodium/calcium exchanger of interest.

Furthermore, it will usually be helpful to have a control for the assay for determining the activity of a sodium/calcium exchanger in response to the addition of a compound in which the compounds are tested in the assay of the invention against cells that preferably are essentially identical to the cells that are used in the assay except that these cells would not express the sodium/calcium exchanger of interest. In this way it can be determined that compounds which are identified by the assay are really exerting their effects through the sodium/calcium exchanger of interest rather than through some unexpected non-specific mechanism. One possibility for such control cells would be to use non-recombinant parent cells where the cells of the actual experiment express the sodium/calcium exchanger of interest.

Other controls for the assay for determining the activity of sodium/calcium exchanger in response to the addition of a compound would be to run the assay without adding a test compound (low control) and to run the assay with a high concentration of test compound (high control).

Other types of controls would involve taking compounds that are identified by the assay of the present invention as agonists or antagonists of sodium/calcium exchangers of interest and testing those compounds in the methods of the prior art in order to confirm that those compounds are also agonists or antagonists when tested in those prior art methods. Furthermore, one skilled in the art would know that it also desirable to run statistical analysis by comparing the assay values to standard values.

The terms "agonist" and "antagonist" refer to receptor effector molecules that modulate signal transduction via a receptor. Receptor effector molecules are capable of binding to the receptor, though not necessarily at the binding site of the natural ligand. Receptor effectors can modulate signal transduction when used alone, i.e. can be surrogate ligands, or can alter signal transduction in the presence of the natural ligand, either to enhance or inhibit signaling by the natural ligand. For example, "antagonists" are molecules that block or decrease the signal transduction activity of receptor, e.g., they can competitively, noncompetitively, and/or allosterically inhibit signal transduction from the receptor, whereas "agonists" potentiate, induce or otherwise enhance the signal transduction activity of a receptor.

The term "disease associated with a sodium/calcium exchanger altered expression" refers to dilated cardiomyopathy, coronary heart disease, arrhythmia, heart failure, etc.

For convenience, the colored substance and other components of the assay may be provided in kits, where the colored substance may be present as a reconstitutable powder or as a cooled solution on ice, in a buffer. The kit may also include buffer, activator, inhibitor, test compound, cells expriming a sodium/calcium exchanger protein, etc.. Cells may be present as lyoplilized cells.

Said kit of parts can be used as a diagnostic kit for diagnosing dilated cardiomyopathy, coronary heart disease, arrhythmia, heart failure, etc.

The following figures and examples shall describe the invention in further details, describing the typical results of the fluorescence based cellular sodium/calcium exchanger assay, without limiting the scope of protection.

EXEMPLIFICATION

**1. Material and methods**

**1.1. Chemicals and equipment**

| Equipment | Manufacturer | Function |
|---|---|---|
| Biomek FX | Beckman Coulter | liquid handling |
| Biomek 2000 | Beckman Coulter | liquid handling |
| 16°C-$CO_2$-Incubator Cytomat | Heraeus | Cell handling |
| $CO_2$-Incubator Hera Cell | Heraeus | Cell handling |
| $FLIPR_{384}$ | Molecular Devices | fluorescent imaging plate reader |

| Materials | Supplier | Cat.No. | Function |
|---|---|---|---|
| CHO-hNCX1 CHO-hNCX2 CHO-hNCX3 | Steinbeis Transfer Zentrum | - | Cell line transfected with human $Na^+$/$Ca^{2+}$ exchanger (NCX1, NCX2, NCX3) |
| A000135933 | ACR | - | NCX1 blocker |
| Fluo4-AM | Molecular Probes | F14201 | Monitoring of intracellular $Ca^{2+}$ |
| Pluronic F-127 | Sigma | P2443 | Detergent |
| Geneticin | Gibco | 10131-027 | Cell selection |
| F-12 (Ham) + L-Glutamin | Gibco | 21765-029 | Cell culture |
| PBS w/o calcium and magnesium | Gibco | 14200-067 | Buffer |
| FCS | PAA | A15-649 | Cell culture |
| Gramicidine | Sigma | G-5002 | Na/K ionophor |

(continued)

| Materials | Supplier | Cat.No. | Function |
|---|---|---|---|
| 96-well PP-microplate, U-Form | Greiner | 650201 | Compound plate |
| 96-well black clear flat bottom plate (TC treated) | Costar | 3904 | Assay plate |
| Trypsin | Biochrom | L2143 | Cell culture |

### 1.2. Cell lines

The recombinant CHO-K1 cell lines stably expressing, respectively, the human NCX1, NCX2 and NCX3 were delivered by *Steinbeis-Transferzentrum für Angewandte Biologische Chemie,* Mannheim.
The cells were kept in continuous culture under standard conditions (37°C, air supplemented with 5% $CO_2$). The CHO-K1 NCX1, CHO-K1 NCX2 and CHO-K1 NCX3 cells were kept in HAM'S-F12 medium plus glutamine supplemented with 10% fetal calf serum (FCS) and 450 μg/ml G418. Cells were passed every 3-4 days after detachment using a Trypsin solution and reseeded with a concentration of 150.000 cells/ml.

### 1.3. Electrophysiological cell line characterization

The electrophysiological characterization was accomplished at longate Biosciences GmbH, Frankfurt.
The cultured cells were detached by application of iced PBS (phosphate buffered saline) or Trypsin at least 18 hours prior to electrophysiological experiments and replated on cover slips. Two bath solutions and pipette solutions were prepared for patch clamp experiments with the following composition:

*Bath (external) solution "low calcium":*

135 mM NMG, 5 mM MgC12, 2 mM CaC12, 4 mM EGTA, 30 mM HEPES, pH 7.4
(HCl → pH 7.0, then CsOH → pH 7.4)

*Bath (external) solution "high calcium":*

139 mM NMG, 5 mM MgCl2, 2 mM CaCl2, 30 mM HEPES, pH 7.4
(HCl → pH 7.0, then CsOH → pH 7.4)

*Pipette (internal) solution:*

100 mM NaCl, 5 mM KCl, 2 mM CaCl2, 2 mM MgCl2, 2.1 mM EGTA, 35 mM TEA-Cl, 4 mM Mg-ATP, 0.5 mM Na-GTP,
5.63 mM Phosphocreatine, 30 mM HEPES, 3.5 U/ml Creatine Phosphokinase (70 ml pipette solution + 3500 U filtered Creatine Phosphokinase, ad 100 ml pipette solution), pH 7.4 (NaOH)
To inhibit NCX currents, 5 mM nickel chloride was dissolved in bath solution "high calcium". NCX activity was measured using the patch clamp technique in the whole cell configuration. The cells were clamped at room temperature (20-25°C) to a holding potential of 0 mV. NCX currents were evoked by application of 2 mM external calcium (bathsolution "high calcium") for 4 seconds (=control). Calcium application was repeated every 20 s. After 5 stimulations, inhibition by nickel was tested using the same protocol but activation with calcium in the presence of 5 mM nickel (5 replicates). Finally, 5 calcium pulses were applied again for washout studies. To characterize NCX currents, peak currents, transported charge and the time constant of signal decay were investigated and analyzed according to the report. All data are presented as mean ± SEM.

### 1.4. Assay reagents

| Reagent | Chemicals | Remarks |
|---|---|---|
| Assay buffer | 133.8 mM NaCl<br>4.7 mM KCl<br>1.25 mM MgCl<br>3.5 mM CaCl2<br>5 mM Glucose<br>10 mM Hepes/NAOH,<br>pH 7.5<br>0,01% Pluronic F-127<br>2.5 mM Probenecid | Probenecid has to be prepared freshly every day |
| Dye loading buffer | Fluo4-AM, 0,02 %<br>Pluronic F-127, 0.1%BSA | 12 $\mu$l Pluronic F-127 (20%) added to 12 $\mu$M Fluo4-AM (2 mM) and then dissolved in assay buffer |
| Gramicidine stock solution | 1 mM gramicidin in Ethanol (4°C) | prepared freshly every day and stored at 4°C until measurement |
| Gramicidine assay solution | 60 $\mu$M gramicidin in assay buffer assay buffer | Plated on PP-plates and stored at 16°C (stable for 3h); 3x concentrated, 20 $\mu$M final concentration; 2% ethanol concentration on cells; |

### 1.5. Final assay procedure

### 1.5.1. Preparation of cell plates

One day prior to the experiment, the cells were detached with Trypsin and plated with a density of 35.000 cells/100 $\mu$l medium/well in 96-well microplates and were incubated for ~22 h at 37°C, 5% $CO_2$ and 90% humidity.

### 1.5.2. Preparation of compound plates

The compounds were prepared in assay buffer with a concentration 1.5x of the final concentration. The compound solution was preincubated and stored at 16°C. For $IC_{50}$ determinations a dilution series with a starting concentration of 45 $\mu$M (final: 30 $\mu$M) was prepared with the Biomek2000.

### 1.5.3. Preparation of gramicidine plates

A 1 mM gramicidine stock solution was prepared every day using cold ethanol (4°C) as solvent. Starting from the stock solution a 60 $\mu$M gramicidine assay solution was prepared using assay buffer. A 96 well polypropylene plate was filled with 150 $\mu$l per well of the gramicidine assay solution and stored at 4°C. The addition plate could only be used two times before refilling was required because the gramicidine solution loses activity very fast at RT.

### 1.5.4. Assay procedure

1. Medium is removed from the cell plates
2. 100 $\mu$l/well of dye loading buffer is added
3. Cells are incubated for 90 min at RT
4. Wash 3x with 100$\mu$l assay buffer per well, discard buffer completely afterwards
5. 80 $\mu$l of test compounds are added at different concentrations (1.5x concentrated) using the Biomek FX
6. Cells are incubated for 45 min at 16°C and 5% $CO_2$ in a $CO_2$ incubator
7. Cell plates are transferred to the FLIPR and recorded by adding 40 $\mu$l of gramicidine assay solution (final concentration: 20 $\mu$M gramicidine) during the measurement

10 $\mu$M of the NCX inhibitor A000135933 is used as high control and buffer as low control.

**1.5.5. FLIPR Setup Parameters**

| FLIPR Experimental Setup Parameters | |
|---|---|
| Exposure | 1.2 sec (at 0.6 W) |
| Aperture | 2.8 |
| Filter | 515-575 ("Calcium") |
| **Defaults (Graphs Setup)** | |
| Subtract bias | From sample 1 |
| Spatial uniformity correction | off |
| Negative control correction | off |
| **First Sequence** | |
| Initial Period | 2 sec |
| Initial Count | 240 (480 sec) |
| Add After Frame | 5 |
| Add Height | 70 $\mu$l |
| Add Speed | 40 $\mu$l/sec |
| Add Volume | 40 $\mu$l |
| Mix | 2x 40 $\mu$l |

**1.6. Data analysis**

The following calculations are based on the data obtained using the statistic export function of the FLIPR software: Statistic 1= max-min (sample1-240). The calculations are corrected for edge effects if necessary.
The inhibition of NCX as primary result refers to the inhibition of the fluorescence increase after addition of gramicidine and is derived from statistic 1. The high control refers to the inhibition of NCX activity with 10 $\mu$M A000135933 and the low control to no NCX inhibition after addition just of buffer. Thus, % inhibition is calculated in reference to the controls (0% inhibition = low control, 100% inhibition = high control). Results are calculated from the corrected raw data and intraplate controls with the following formula:

$$\text{Inhibition}\,(\%) = 100 \circ \left( 1 - \frac{(\text{sample} - \text{low control})}{(\text{high control} - \text{low control})} \right)$$

**2. Results**

**2.1. Electrophysiological cell line characterisation**

**2.1.1. Parental cell line**

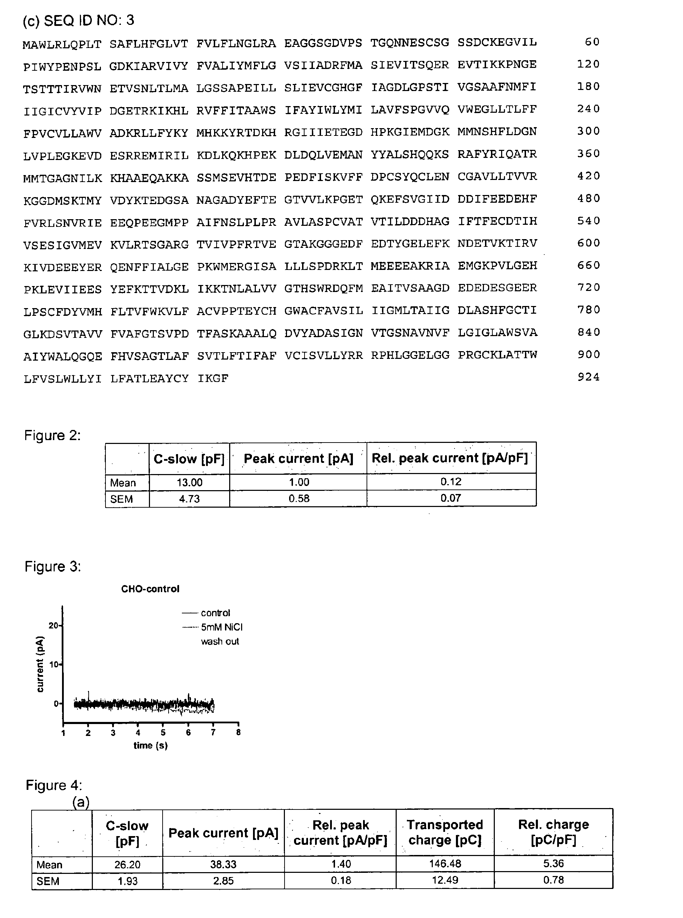

The electrophysiological data were obtained from longate GmbH. CHO-K1 cells were used to establish the stable CHO-NCX1, CHO-NCX2 and CHO-NCX3 cell lines. The parental cells exhibited no significant current response ($0.12 \pm 0.07$ pA/pF, figure 2) when extracellular $Ca^{2+}$ was applied under high intracellular $Na^+$ concentrations. The use of nickel did not produce artifacts under the described conditions. Typical currents are shown in figure 3.

### 2.1.2. CHO-NCX cell lines

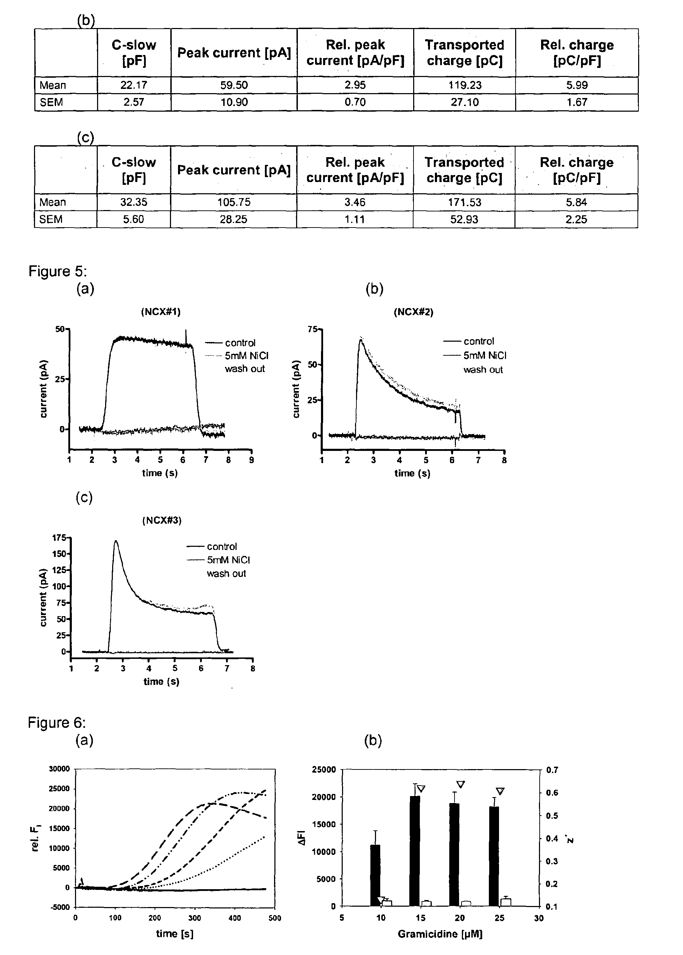

The CHO-NCX1 cells revealed outward steady-state currents after $Ca^{2+}$ application with almost no deactivation after four seconds. The relative peak current was $1.40 \pm 0.18$ pA/pF (n=3, figure 4a). Currents could be completely and reversible blocked by application of 5 mM nickel ($91.3 \pm 3.3$ %).
The CHO-NCX2 cells revealed outward currents under reverse mode conditions. The relative peak current was $2.95 \pm 0.70$ pA/pF (n=6, figure 4b). Currents decayed with a time constant of $1.26 \pm 0.11$ s and could be completely and reversible blocked by application of 5 mM nickel (washout: $100.2 \pm 1.9$ %).
The CHO-NCX3 cells revealed outward currents under reverse mode conditions decaying with a time constant of $0.80 \pm 0.08$ s. The relative peak current was $3.46 \pm 1.11$ pA/pF (n=4, figure 4c). Currents could be completely and reversible blocked by application of 5 mM nickel (washout: $111.5 \pm 2.4$ %). Typical currents are shown in figure 5.

### 2.2. Assay parameter

### 2.2.1. NCX1 assay

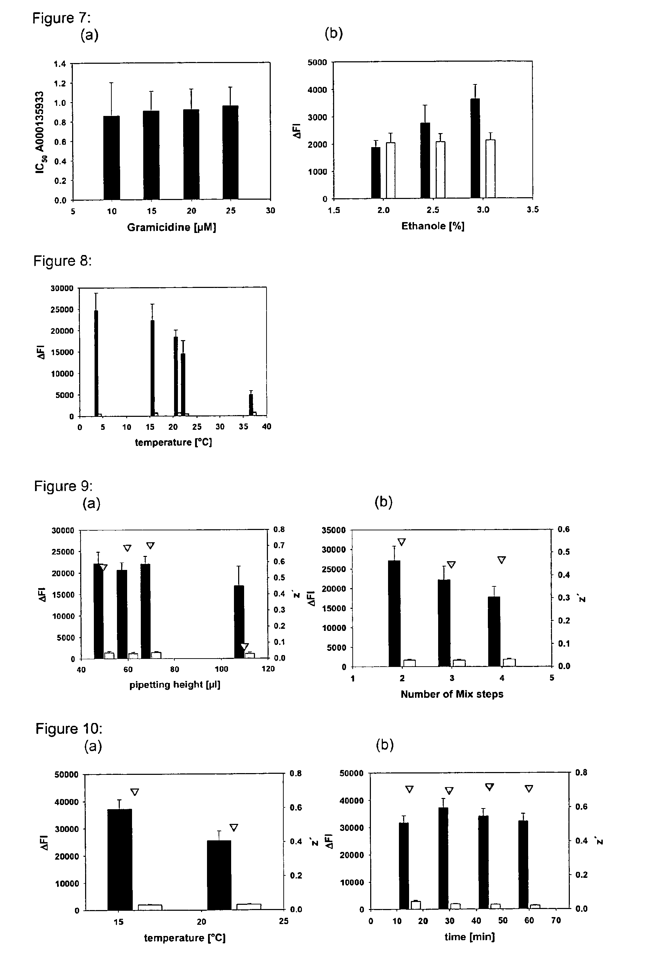

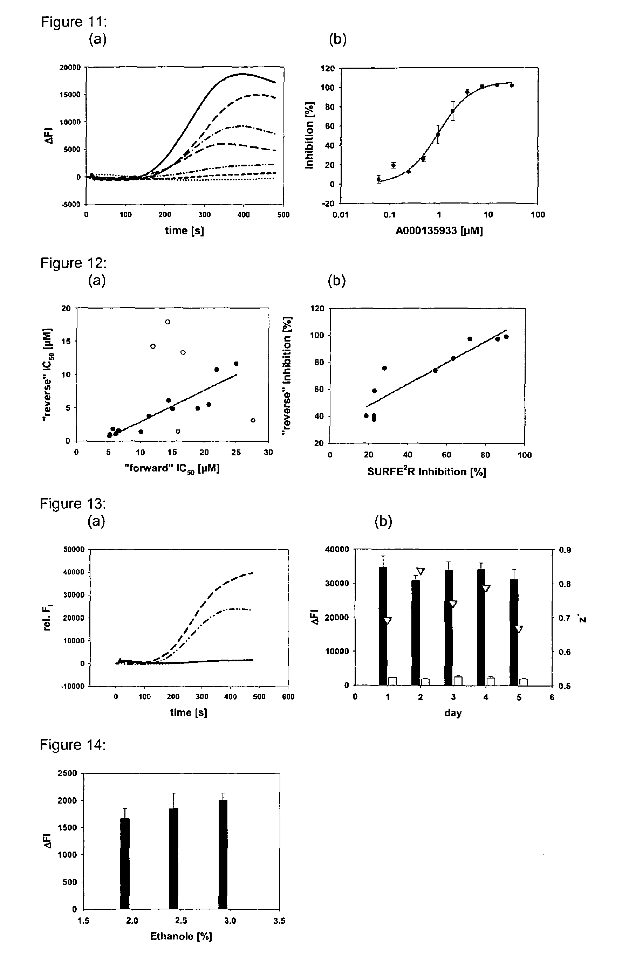

HTS-based NCX1 assays analysing the "reverse" mode were not known in the literature. The application of gramicidine is known out of combined patch clamp and fluorescence measurements. All parameters had to be optimized. By reason of flexibility between the "forward" and the "reverse" mode assay, running at the same time, the buffer conditions (e.g. $Ca^{2+}$ concentration, pH) and cell number per well were adapted from the "forward" mode assay and showed very good results.
In the first experiments the influence of the gramicidine concentration and storage on the $Ca^{2+}$-import activity of NCX1 were investigated (Fig. 6a,b).
A gramicidine concentration of 20 $\mu$M showed the best results on the $Ca^{2+}$-import activity regarding S/B and z'-factor. Lower concentrations were not sufficient due to slow kinetics and higher concentrations caused cellular side effects (Fig. 6a,b). The $IC_{50}$ of the tool compound A000135933 (also Figure 11a,b) was unaffected by different gramicidine concentrations (Fig. 7a). Gramicidine was solved in ethyl alcohol and the influence of the solvent had to be characterized. Up to 2 % ethyl alcohol showed no effect on the background fluorescence of the assay while increasing concentrations led to a slight increase in fluorescence (Fig. 7b).
The preparation of the gramicidine solution and the storage was a critical step, because gramicidine is degraded at higher temperatures. In the following experiments gramicidine was solved and stored at different temperatures. The gramicidine solution had to be prepared and stored at 4°C otherwise gramicidine was degraded very fast (Figure 8).
Different FLIPR settings concerning pipette height, presoak and mixing were checked to improve the S/B and z'-factor (Figure 9a,b). The best z'factor was achieved with a pipette height of 70 $\mu$l with two mixing cycles.
Using the parameters described above two temperatures (16 and 22°C) were checked for compound incubation (Figure 10a). The best results were obtained with 16°C and the best incubation time regarding S/B, z' and $IC_{50}$ (A000135933) results was 45 min (Figure 10b).
One of the key requirements of the "reverse" mode assay was the sensitivity of compounds. The tool compound for the NCX1 project is A000135933, an iminothiazole derived out of the screen with the "forward" mode assay. The compound has an $IC_{50}$ of 5.9 $\mu$M in the "forward" assay and 1.44 $\mu$M in patch clamp experiments. The compound is in use as standard control in the "forward" assay. To examine the effect, A000135933 was tested under different gramicidine concentrations in the "reverse" assay. Typical inhibitory effects on the NCX1 activity after activation with 20 $\mu$M gramicidine and the calculated dose response relationship is shown in Figure 11 a and b.
The NCX1 cell line was stable concerning S/B and $IC_{50}$ of the standard (A000135933) for two month. The effect of the tool compound A000135933 on the $Ca^{2+}$-import activity of NCX1 is shown in Figure 11 a and the $IC_{50}$ was around 1 $\mu$M (Figure 11b). The z' values were between 0.7 and 0.8. To compare this assay ("reverse") with the fluorescence based cellular $Ca^{2+}$-export mode assay ("forward") and with an electrophysiological technique called SURFE$^2$R (longate Biosciences, Frankfurt) the $IC_{50}$ of 18 or the Inhibition at 10 $\mu$M of 11 compounds were tested respectively (Fig. 12a,b).
The "reverse" assay was in general 3-5 fold more sensitive than the "forward" assay (Fig. 12a, black dots) and the data correlates with a $r^2$ of 0.86. Some compounds do not show a significant increase in inhibition (Fig. 8a, red dots). Other compounds inhibit the NCX1 with a much higher potency (Fig. 8a, blue dots) perhaps indicating a transport-mode specific effect. The percent Inhibition of the compounds at 10 $\mu$M derived from the "reverse" assay was higher (up to 20%) than the SURFE$^2$R values (Fig. 12b), but the correlation with the SURFE$^2$R-technique was good with a $r^2$ of 0.83.

### 2.2.2. NCX2 assay

No NCX2 assays analysing the "forward" or "reverse" mode were known in the literature. The establishment of the "forward mode" assay was not successful due to limitations in the sensitivity and robustness of the assay. For clarity - no data is shown in this report. The "reverse mode" assay is based on the application of gramicidin. Gramicidin forms alkali-permeable pores. Gramicidin-induced $Ca^{2+}$ influx is indirect and NCX dependent. The activation of NCX1 after gramicidin addition is known from radioactive ion flux or fluorescence measurements on primary neurons (Kiedrowski et al., Journal of Neurochemistryl, 2004). By reason of comparability between the NCX assays of the different subtypes, running at the same time, the buffer conditions (e.g. $Ca^{2+}$ concentration, pH), cell number, incubation times and gramicidin concentrations were adapted for the NCX2 assay from the "reverse" mode NCX1 assay.

In the first experiments the buffer conditions, incubation times, gramicidin concentration and storage were checked on the $Ca^{2+}$-import activity of NCX2 (Fig. 13a,b).

The kinetic of the fluorescence increase is a little bit slower for NCX2 compared to NCX1. The measurement time had to be extended to 10 min. The buffer system, cell number, incubation times, gramicidin concentration (20 $\mu$M) and storage showed very good results. These parameters must not be further optimized. Gramicidin was solved in ethyl alcohol and the influence of the solvent had to be characterized. Up to 3 % ethyl alcohol showed no effect on the background fluorescence of the assay (Fig. 14).

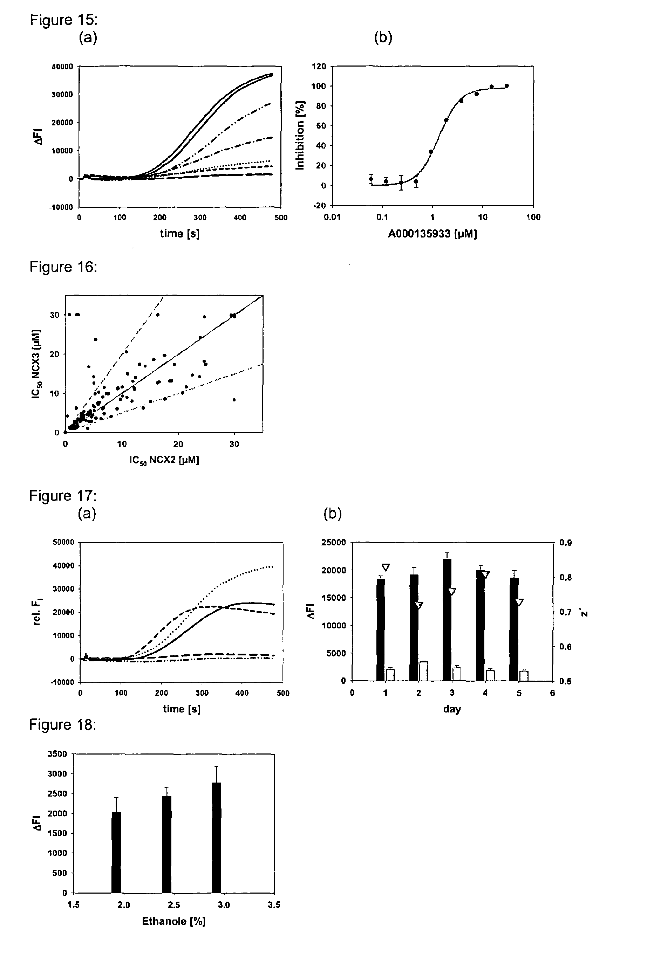

One of the key requirements of the "reverse" mode assay was the sensitivity to compounds but there were no electrophysiological data available for the tool compounds on NCX2. The main tool compound for the NCX1 project is A000135933, an iminothiazole derived out of the NCX1-screen. The compound has an $IC_{50}$ on NCX1 of 1.8 $\mu$M in the "reverse" assay and 1.44 $\mu$M in patch clamp experiments. The effect of A000135933 after activation with 20 $\mu$M gramicidin on the NCX2 activity was examined (Fig 16a). The calculated dose response relationship is shown in Figure 15b.

The NCX2 cell line was stable concerning S/B and $IC_{50}$ of the standard (A000135933) for two month. The effect of the tool compound A000135933 on the $Ca^{2+}$-import activity of NCX2 is shown in Figure 15a and the $IC_{50}$ (1.25 $\pm$ 0.19 $\mu$M, n=26, example Fig. 15b) was a little bit lower on NCX2 than on NCX1 (1.78 $\pm$ 1.03 $\mu$M, n=55) indicating that the compound is more potent for NCX2. The z' values were between 0.75 and 0.85. 125 compounds from the NCX1 project were tested with the NCX2 "reverse mode" assay and compared with the NCX1 data. The results are summarized in Figure 16.

Nearly all compounds with an effect on the NCX1 activity also inhibited NCX2 (Figure 16). A lot of compounds inhibited NCX2 with a more than two-fold higher potency than NCX1. Only 4 compounds showed a more than two-fold selectivity for NCX1. These data indicates that there is a possibility to obtain NCX1 selective compounds.

### 2.2.3. NCX3 assay

NCX3 assays were not known in the literature. The establishment of the "forward mode" assay was not successful due to limitations in the sensitivity and robustness of the assay. For clarity - no data is shown in this report. The "reverse mode" assay is based on the application of gramicidin. Gramicidin forms alkali-permeable pores. Gramicidin-induced $Ca^{2+}$ influx is indirect and NCX dependent. The activation of NCX1 after gramicidin addition is known from radioactive ion flux or fluorescence measurements on primary neurons (Kiedrowski et al., Journal of Neurochemistry, 2004). By reason of comparability between the NCX assays of the different subtypes, running at the same time, the buffer conditions (e.g. $Ca^{2+}$ concentration, pH), cell number, incubation times and gramicidin concentrations were adapted for the NCX3 assay from the "reverse" mode NCX1 assay.

In the first experiments the buffer conditions, cell number, incubation times, gramicidin concentration and storage were checked on the $Ca^{2+}$-import activity of NCX3 (Fig. 18a,b).

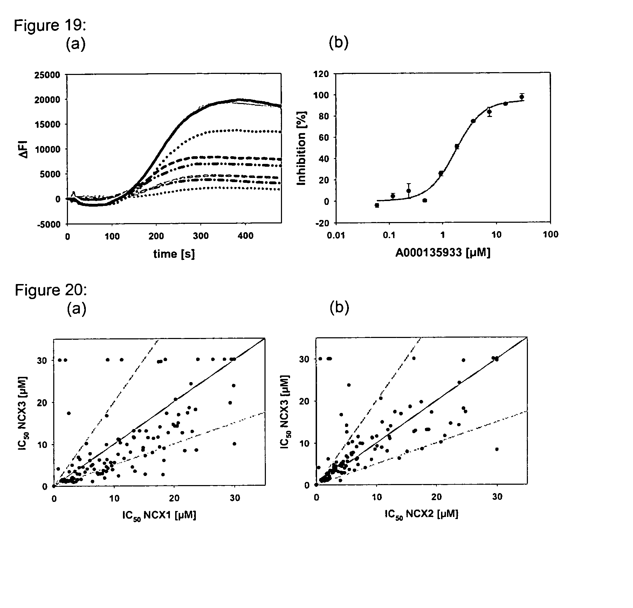

The kinetic of the fluorescence increase is a faster in comparison to NCX1 and NCX2. The buffer system, incubation times, gramicidin concentration (20 $\mu$M) and storage showed very good results. These parameters must not be further optimized. Gramicidin was solved in ethyl alcohol and the influence of the solvent had to be characterized. Up to 2 % ethyl alcohol showed no effect on the background fluorescence of the assay (Fig. 19) more than 2% leads to slight increase in background fluorescence.

One of the key requirements of the "reverse" mode assay was the sensitivity to compounds but there were no electrophysiological data available for the tool compounds on NCX3. The main tool compound for the NCX1 project is A000135933, an iminothiazole derived out of the NCX1-screen. The compound has an $IC_{50}$ on NCX1 of 1.8 $\mu$M in the "reverse" assay and 1.44 $\mu$M in patch clamp experiments. The IC50 on NCX2 is 1.25 $\mu$M in the "reverse" assay. The effect of A000135933 after activation with 20 $\mu$M gramicidin on the NCX3 activity was examined (Fig 21a) and the calculated dose response relationship is shown in Figure 19b.

The NCX3 cell line was stable concerning S/B and $IC_{50}$ of the standard (A000135933) for two month. The effect of the tool compound A000135933 on the $Ca^{2+}$-import activity of NCX3 is shown in Figure 19a and the $IC_{50}$ was 1.63 $\pm$ 0.30 $\mu$M (n=23, example Fig. 19b). These data indicates that the tool compound is not selective for a specific subtype ($IC_{50}$ NCX1: 1.78 $\pm$ 1.03; $IC_{50}$ NCX2: 1.25 $\pm$ 0.19). The z' values were between 0.70 and 0.85. 125 compounds from the NCX1 project were tested with the NCX3 "reverse mode" assay and compared with the NCX1 and NCX2 data. The results are summarized in Figure 20a and 20b.

Nearly all compounds with an effect on the NCX1 activity also inhibited NCX3 (Figure 20a). A lot of compounds inhibited NCX3 with a more than two-fold higher potency than NCX1. Six compounds showed a more than two-fold selectivity for NCX1. In comparison to NCX2 - nine compounds were more potent on NCX2 and two substances on NCX3 (Figure 20b). These data indicates that there is a possibility to obtain NCX subtype selective compounds.

DESCRIPTION OF THE FIGURES

Figure 1:

(a) Amino acid sequence of NCX1 represented by SEQ ID NO: 1;
(b) Amino acid sequence of NCX2 represented by SEQ ID NO: 2;
(c) Amino acid sequence of NCX3 represented by SEQ ID NO: 3.

Figure 2:

Peak currents of CHO-K1 cells after addition of 2 mM $Ca^{2+}$ (n = 3).

Figure 3:

Typical current of CHO-K1 cells after addition of 2 mM $Ca^{2+}$ and application of 5 mM nickel.

Figure 4:

(a) Peak currents of CHO-NCX1 cells after addition of 2 mM $Ca^{2+}$ (n = 3);
(b) Peak currents of CHO-NCX2 cells after addition of 2 mM $Ca^{2+}$ (n = 6);
(c) Peak currents of CHO-NCX3 cells after addition of 2 mM $Ca^{2+}$ (n = 6).

Figure 5:

(a) Typical current of CHO-NCX1 cells after addition of 2 mM $Ca^{2+}$, application of 5 mM nickel and washout;
(b) Typical current of CHO-NCX2 cells after addition of 2 mM $Ca^{2+}$, application of 5 mM nickel and washout;
(c) Typical current of CHO-NCX3 cells after addition of 2 mM $Ca^{2+}$, application of 5 mM nickel and washout.

Figure 6:

(a) Effect of different concentrations of gramicidine (- 0 $\mu$M, ·····10 $\mu$M, - - -15 $\mu$M, --- 20 $\mu$M and --25 $\mu$M) on the $Ca^{2+}$-import activity of NCX1. The effect of the addition of the NCX1 inhibitor A000135933 is shown at 20 $\mu$M gramicidine (·- ·-);
(b) Effect of different concentrations of gramicidine (10 $\mu$M, 15 $\mu$M, 20 $\mu$M and 25 $\mu$M) on the $Ca^{2+}$-import activity of NCX1 without (■, high control) and with 10 $\mu$M (□, low control) of the NCX1 inhibitor A000135933. The z'-factor is indicated as $\nabla$.

Figure 7:

(a) Effect of different concentrations of gramicidine (10 $\mu$M, 15 $\mu$M, 20 $\mu$M and 25 $\mu$M) on the $IC_{50}$ (■) of the NCX1 inhibitor A000135933;
(b) Effect of different concentrations of ethyl alcohol on the Fluo4-fluorescence of the CHO-NCX1 cells (■) and with the NCX inhibitor A000135933 (□) without gramicidine stimulation.

Figure 8:

Effect of 20 $\mu$M gramicidine solved and stored at different temperatures on the $Ca^{2+}$-import activity of NCX1 without (■, high control) and with 10 $\mu$M (□, low control) of the NCX1 inhibitor A000135933.

Figure 9:

(a) High controls (■, buffer), low controls (□, 10 $\mu$M A000135933) and z'-factor ($\nabla$) at different pipette heights;

(b) High controls (■, buffer), low controls (□, 10 $\mu$M A000135933) and z'-factor ($\nabla$) with different numbers of mixing cycles.

Figure 10:

(a) High controls (■, buffer), low controls (□, 10 $\mu$M A000135933) and z'-factor ($\nabla$) with different temperatures during compound incuba-tion (30 min);
(b) High controls (■, buffer), low controls (□, 10 $\mu$M A000135933) and z'-factor ($\nabla$) with different compound incubation times at 16°C.

Figure 11:

(a) Effect of different concentrations of A000135933 ($\cdots$30 $\mu$M, - - -15 $\mu$M, $\cdot\cdot-\cdot\cdot$7.5 $\mu$M,--3.75 $\mu$M, $\cdot\cdot-\cdot\cdot$-1.88 $\mu$M, - - -0.94 $\mu$M, -0.5 $\mu$M) on the $Ca^{2+}$-import activity of NCX1 after addition of 20 $\mu$M gramicidine (Incubation: 22°C, 30 min);
(b) Dose response relationship of A000135933. The $IC_{50}$ value is 0.97 $\mu$M.

Figure 12:

(a) Correlation of the $IC_{50}$ of 18 compounds tested with the fluorescence based cellular $Ca^{2+}$-export ("forward") and $Ca^{2+}$-import ("reverse") assay;
(b) Correlation of the %-Inhibition at 10 $\mu$M of 11 compounds tested with the fluorescence based cellular $Ca^{2+}$-import assay ("reverse") and the electrophysiological SURFE$^2$R technique.

Figure 13:

(a) $Ca^{2+}$-import activity of NCX2 without (-) and with 20 $\mu$M gramicidin (---) in comparison to NCX1 ($\cdot\cdot-\cdot\cdot$). The effect of the addition of the NCX inhibitor A000135933 on NCX2 activity is shown at 20 $\mu$M gramicidin ($\cdots$);
(b) Effect of 20 $\mu$M gramicidin on the $Ca^{2+}$-import activity of NCX2 at 5 days without (■, high control) and with 10 $\mu$M (□, low control) of the NCX1 inhibitor A000135933. The z'-factor is indicated as $\nabla$.

Figure 14:

Effect of different concentrations of ethyl alcohol on the Fluo4-fluorescence of the CHO-NCX2 cells (■) without gramicidin stimulation.

Figure 15:

(a) Effect of different concentrations of A000135933 (- -30 $\mu$M, - - - 15 $\mu$M, - - -7.5 $\mu$M, $\cdots$3.75 $\mu$M, $\cdot\cdot-\cdot\cdot$-1.88 $\mu$M, $\cdot\cdot-\cdot\cdot$-0.94 $\mu$M, -0.5 $\mu$M and -0 $\mu$M) on the $Ca^{2+}$-import activity of NCX2 after addition of 20 $\mu$M gramicidin (Incubation: 22°C, 45 min);
(b) Dose response relationship of A000135933. The $IC_{50}$ value of this example is 1.4 $\mu$M.

Figure 16:

Comparison of the $IC_{50}$ of 125 compounds on NCX2 and NCX1. Compounds were tested with the fluorescence based cellular $Ca^{2+}$-import assay ("reverse"). The dark grey line (-) marks the line of equal potency and the light grey dashed line (- - -) indicates a two-fold split in potency on a particular subtype.

Figure 17:

(a) $Ca^{2+}$-import activity of NCX3 without ($\cdot\cdot-\cdot\cdot$) and with 20 $\mu$M gramicidin (- - -) in comparison to NCX1 (-) and NCX2 ($\cdots$). The effect of the addition of the NCX inhibitor A000135933 on NCX3 activity is shown at 20 $\mu$M gramicidin (- -);
(b) Effect of 20 $\mu$M gramicidin on the $Ca^{2+}$-import activity of NCX3 at 5 days without (■, high control) and with 10 $\mu$M (□, low control) of the NCX inhibitor A000135933. The z'-factor is indicated as $\nabla$.

Figure 18:

Effect of different concentrations of ethyl alcohol on the Fluo4-fluorescence of the CHO-NCX3 cells (■) without gramicidin stimulation.

Figure 19:

(a) Effect of different concentrations of A000135933 (·····30 $\mu$M, ·-·-·-15 $\mu$M, - - -7.5 $\mu$M, ··-··-3.75 $\mu$M, - - -1.88 $\mu$M, ·····0.94 $\mu$M, -0.5 $\mu$M and -0 $\mu$M) on the Ca$^{2+}$-import activity of NCX3 after addition of 20 $\mu$M gramicidin (Incubation: 22°C, 45 min);
(b) Dose response relationship of A000135933. The IC$_{50}$ value for this example is 1.76 $\mu$M.

Figure 20:

(a) Comparison of the IC$_{50}$ of 125 compounds on NCX3 and NCX1. Compounds were tested with the fluorescence based cellular Ca$^{2+}$-import assay ("reverse"). The dark grey line (-) marks the line of equal potency and the light grey dashed line (- - -) indicates a two-fold split in potency on a particular subtype;
(b) Comparison of the IC$_{50}$ of 125 compounds on NCX3 and NCX2. Compounds were tested with the fluorescence based cellular Ca$^{2+}$-import assay ("reverse"). The dark grey line (-) marks the line of equal potency and the light grey dashed line (- - -) indicates a two-fold split in potency on a particular subtype.

Application Project
-----------------
<110> Sanofi-Aventis

<120> Title : Fluorescence based assay to detect sodium-calcium exchanger (NCX)
              "forward mode" modulating compounds
<130> AppFileReference : DE2008/024

<140> CurrentAppNumber :

<141> CurrentFilingDate :

<160> 3

Sequence
---------
<213> OrganismName : Homo sapiens
<400> PreSequenceString :

```
MYNMRRLSLS PTFSMGFHLL VTVSLLFSHV DHVIAETEME GEGNETGECT GSYYCKKGVI      60
LPIWEPQDPS FGDKIARATV YFVAMVYMFL GVSIIADRFM SSIEVITSQE KEITIKKPNG     120
ETTKTTVRIW NETVSNLTLM ALGSSAPEIL LSVIEVCGHN FTAGDLGPST IVGSAAFNMF     180
IIIALCVYVV PDGETRKIKH LRVFFVTAAW SIFAYTWLYI ILSVISPGVV EVWEGLLTFF     240
FFPICVVFAW VADRRLLFYK YVYKRYRAGK QRGMIIEHEG DRPSSKTEIE MDGKVVNSHV     300
ENFLDGALVL EVDERDQDDE EARREMARIL KELKQKHPDK EIEQLIELAN YQVLSQQQKS     360
RAFYRIQATR LMTGAGNILK RHAADQARKA VSMHEVNTEV TENDPVSKIF FEQGTYQCLE     420
NCGTVALTII RRGGDLTNTV FVDFRTEDGT ANAGSDYEFT EGTVVFKPGD TQKEIRVGII     480
DDDIFEEDEN FLVHLSNVKV SSEASEDGIL EANHVSTLAC LGSPSTATVT IFDDDHAGIF     540
TFEEPVTHVS ESIGIMEVKV LRTSGARGNV IVPYKTIEGT ARGGGEDFED TCGELEFQND     600
EIVKTISVKV IDDEEYEKNK TFFLEIGEPR LVEMSEKKAL LLNELGGFTI TGKYLFGQPV     660
FRKVHAREHP ILSTVITIAD EYDDKQPLTS KEEEERRIAE MGRPILGEHT KLEVIIEESY     720
EFKSTVDKLI KKTNLALVVG TNSWREQFIE AITVSAGEDD DDDECGEEKL PSCFDYVMHF     780
LTVFWKVLFA FVPPTEYWNG WACFIVSILM IGLLTAFIGD LASHFGCTIG LKDSVTAVVF     840
VALGTSVPDT FASKVAATQD QYADASIGNV TGSNAVNVFL GIGVAWSIAA IYHAANGEQF     900
KVSPGTLAFS VTLFTIFAFI NVGVLLYRRR PEIGGELGGP RTAKLLTSCL FVLLWLLYIF     960
FSSLEAYCHI KGF                                                       973
```
<212> Type : PRT
<211> Length : 973
       SequenceName : SEQ ID NO 1
       SequenceDescription :

Sequence
---------
<213> OrganismName : Homo sapiens
<400> PreSequenceString :

```
MAPLALVGVT LLLAAPPCSG AATPTPSLPP PPANDSDTST GGCQGSYRCQ PGVLLPVWEP      60
DDPSLGDKAA RAVVYFVAMV YMFLGVSIIA DRFMAAIEVI TSKEKEITIT KANGETSVGT     120
VRIWNETVSN LTLMALGSSA PEILLSVIEV CGHNFQAGEL GPGTIVGSAA FNMFVVIAVC     180
IYVIPAGESR KIKHLRVFFV TASWSIFAYV WLYLILAVFS PGVVQVWEAL LTLVFFPVCV     240
VFAWMADKRL LFYKYVYKRY RTDPRSGIII GAEGDPPKSI ELDGTFVGAE APGELGGLGP     300
GPAEARELDA SRREVIQILK DLKQKHPDKD LEQLVGIANY YALLHQQKSR AFYRIQATRL     360
MTGAGNVLRR HAADASRRAA PAEGAGEDED DGASRIFFEP SLYHCLENCG SVLLSVTCQG     420
GEGNSTFYVD YRTEDGSAKA GSDYEYSEGT LVFKPGETQK ELRIGIIDDD IFEEDEHFFV     480
RLLNLRVGDA QGMFEPDGGG RPKGRLVAPL LATVTILDDD HAGIFSFQDR LLHVSECMGT     540
VDVRVVRSSG ARGTVRLPYR TVDGTARGGG VHYEDACGEL EFGDDETMKT LQVKIVDDEE     600
YEKKDNFFIE LGQPQWLKRG ISALLLNQGD GDRKLTAEEE EARRIAEMGK PVLGENCRLE     660
VIIEESYDFK NLALVIGTHS WREQFLEAIT VSAGDEEEEE DGSREERLPS     720
CFDYVMHFLT VFWKVLFACV PPTEYCHGWA CFGVSILVIG LLTALIGDLA SHFGCTVGLK     780
DSVNAVVFVA LGTSIPDTFA SKVAALQDQC ADASIGNVTG SNAVNVFLGL GVAWSVAAVY     840
WAVQGRPFEV RTGTLAFSVT LFTVFAFVGI AVLLYRRRPH IGGELGGPRG PKLATTALFL     900
GLWLLYILFA SLEAYCHIRG F                                               921
```
<212> Type : PRT
<211> Length : 921
       SequenceName : SEQ ID NO 2
       SequenceDescription :

Sequence
---------
<213> OrganismName : Homo sapiens

```
<400> PreSequenceString :
MAWLRLQPLT SAFLHFGLVT FVLFLNGLRA EAGGSGDVPS TGQNNESCSG SSDCKEGVIL        60
PIWYPENPSL GDKIARVIVY FVALIYMFLG VSIIADRFMA SIEVITSQER EVTIKKPNGE        120
TSTTTIRVWN ETVSNLTLMA LGSSAPEILL SLIEVCGHGF IAGDLGPSTI VGSAAFNMFI        180
IIGICVYVIP DGETRKIKHL RVFFITAAWS IFAYIWLYMI LAVFSPGVVQ VWEGLLTLFF        240
FPVCVLLAWV ADKRLLFYKY MHKKYRTDKH RGIIIETEGD HPKGIEMDGK MMNSHFLDGN        300
LVPLEGKEVD ESRREMIRIL KDLKQKHPEK DLDQLVEMAN YYALSHQQKS RAFYRIQATR        360
MMTGAGNILK KHAAEQAKKA SSMSEVHTDE PEDFISKVFF DPCSYQCLEN CGAVLLTVVR        420
KGGDMSKTMY VDYKTEDGSA NAGADYEFTE GTVVLKPGET QKEFSVGIID DDIFEEDEHF        480
FVRLSNVRIE EEQPEEGMPP AIFNSLPLPR AVLASPCVAT VTILDDDHAG IFTFECDTIH        540
VSESIGVMEV KVLRTSGARG TVIVPFRTVE GTAKGGGEDF EDTYGELEFK NDETVKTIRV        600
KIVDEEEYER QENFFIALGE PKWMERGISA LLLSPDRKLT MEEEEAKRIA EMGKPVLGEH        660
PKLEVIIEES YEFKTTVDKL IKKTNLALVV GTHSWRDQFM EAITVSAAGD EDEDESGEER        720
LPSCFDYVMH FLTVFWKVLF ACVPPTEYCH GWACFAVSIL IIGMLTAIIG DLASHFGCTI        780
GLKDSVTAVV FVAFGTSVPD TFASKAAALQ DVYADASIGN VTGSNAVNVF LGIGLAWSVA        840
AIYWALQGQE FHVSAGTLAF SVTLFTIFAF VCISVLLYRR RPHLGGELGG PRGCKLATTW        900
LFVSLWLLYI LFATLEAYCY IKGF                                                924
<212> Type : PRT
<211> Length : 924
     SequenceName : SEQ ID NO 3
     SequenceDescription :
```

## Claims

1. An assay for determining the calcium import activity of a sodium/calcium exchanger comprising:

    a) providing cells expressing a sodium/calcium exchanger;
    b) providing a colored substance for determining intracellular calcium;
    c) contacting cells with a sodium/calcium exchanger activator; and
    d) comparing the calcium mediated change in the luminescent signal from said colored substance to a luminescent signal produced in a control experiment.

2. The assay according to claim 1, wherein the sodium/calcium exchanger is a NCX protein of the following list: NCX1, NCX2, NCX3; or a NCKX protein of the following list: NCKX1, NCKX2, NCKX3, NCKX4, NCKX5.

3. The assay according to claim 1, wherein the sodium/calcium exchanger is a NCX protein of the following list: NCX1, NCX2, NCX3.

4. The assay according to claims 1 to 3, wherein the sodium/calcium exchanger is of mammalian origin, preferably from rat, mouse, dog, bovine, pig, ape or human.

5. The assay according to claims 1 to 4, wherein the cells are selected from the group consisting of: CHO, HEK, COS7 and JURKAT cells.

6. The assay according to claims 1 to 5, wherein said colored substance is added to the cells as a dye precursor capable of entering the cells and being hydrolyzed to a dye, whereby the dye complexes with calcium in said cells and provides a luminescent signal.

7. The assay according to claims 1 to 6, wherein said luminescent signal is fluorescence and said monitoring step c) employs a FLIPR device.

8. The assay according to claim 6, wherein said dye precursor is an acetoxymethylester derivate.

9. The assay according to claim 6, wherein said dye is the calcium sensitive fluorescence dye fluo-4.

10. Use of the assay according to claims 1 to 9 to test a compound for activity as an agonist or antagonist of the calcium import activity of a sodium/calcium exchanger.

11. Use of the assay according to claims 1 to 9 for the diagnosis of a disease associated with a sodium/calcium exchanger altered expression.

**12.** An assay for determining the calcium import activity of a sodium/calcium exchanger in response to the addition of a compound comprising:

   a) providing cells expressing a sodium/calcium exchanger;
   b) providing a colored substance for determining intracellular calcium;
   c) contacting cells with a compound, wherein said cells have been treated, prior to treating with said compound, with a sodium/calcium exchanger activator; and
   d) comparing the calcium mediated change in the luminescent signal from said colored substance to a luminescent signal produced in a control experiment.

**13.** The assay according to claim 12, wherein the sodium/calcium exchanger is a NCX protein of the following list: NCX1, NCX2, NCX3; or a NCKX protein of the following list: NCKX1, NCKX2, NCKX3, NCKX4, NCKX5.

**14.** The assay according to claim 12, wherein the sodium/calcium exchanger is a NCX protein of the following list: NCX1, NCX2, NCX3.

**15.** The assay according to claims 12 to 14, wherein the sodium/calcium exchanger is of mammalian origin, preferably from rat, mouse, dog, bovine, pig, ape or human.

**16.** The assay according to claims 12 to 15, wherein the cells are selected from the group consisting of: CHO, HEK, COS7 and JURKAT cells.

**17.** The assay according to claims 12 to 16, wherein said colored substance is added to the cells as a dye precursor capable of entering the cells and being hydrolyzed to a dye, whereby the dye complexes with calcium in said cells and provides a luminescent signal.

**18.** The assay according to claims 12 to 17, wherein said luminescent signal is fluorescence and said monitoring step c) employs a FLIPR device.

**19.** The assay according to claim 17, wherein said dye precursor is an acetoxymethylester derivate.

**20.** The assay according to claim 17, wherein said dye is the calcium sensitive fluorescence dye fluo-4.

**21.** The assay according to claims 12 to 20, wherein said compound is a sodium/calcium exchanger antagonist.

**22.** A kit of parts comprising:

   a) lyophilized cells expriming a sodium/calcium exchanger;
   b) a colored substance;
   c) a compound buffer; and
   d) a colored substance buffer.

**23.** The kit of parts according to claim 22, wherein said colored substance is the calcium sensitive fluorescence dye fluo-4.

**24.** The kit of parts according to claims 22 and 23, wherein the sodium/calcium exchanger is a NCX protein of the following list: NCX1, NCX2, NCX3; or a NCKX protein of the following list: NCKX1, NCKX2, NCKX3, NCKX4, NCKX5.

**25.** The kit of parts according to claims 22 and 23, wherein the sodium/calcium exchanger is a NCX protein of the following list: NCX1, NCX2, NCX3.

**26.** The kit of parts according to claims 22 to 25, wherein the sodium/calcium exchanger is of mammalian origin, preferably from rat, mouse, dog, bovine, pig, ape or human.

**27.** Use of a kit of parts according to claims 22 to 26 to test a compound for activity as an agonist or antagonist of the calcium import activity of a sodium/calcium exchanger.

**28.** Use of a kit of parts according to claims 22 to 26 for the diagnosis of a disease associated with a sodium/calcium exchanger altered expression.

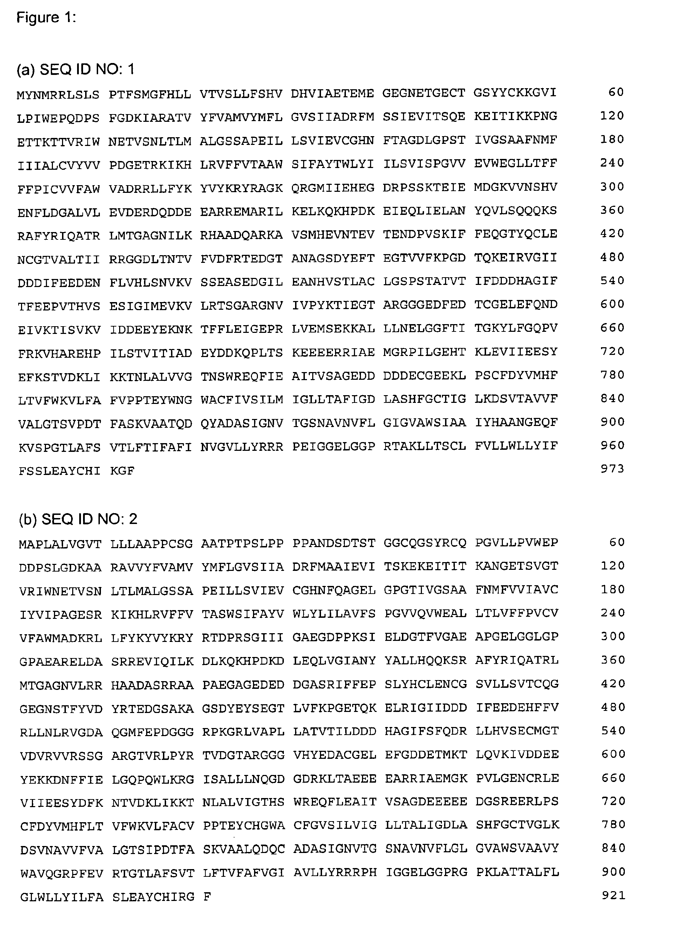

Figure 1:

(a) SEQ ID NO: 1

```
MYNMRRLSLS PTFSMGFHLL VTVSLLFSHV DHVIAETEME GEGNETGECT GSYYCKKGVI        60
LPIWEPQDPS FGDKIARATV YFVAMVYMFL GVSIIADRFM SSIEVITSQE KEITIKKPNG       120
ETTKTTVRIW NETVSNLTLM ALGSSAPEIL LSVIEVCGHN FTAGDLGPST IVGSAAFNMF       180
IIIALCVYVV PDGETRKIKH LRVFFVTAAW SIFAYTWLYI ILSVISPGVV EVWEGLLTFF       240
FFPICVVFAW VADRRLLFYK YVYKRYRAGK QRGMIIEHEG DRPSSKTEIE MDGKVVNSHV       300
ENFLDGALVL EVDERDQDDE EARREMARIL KELKQKHPDK EIEQLIELAN YQVLSQQQKS       360
RAFYRIQATR LMTGAGNILK RHAADQARKA VSMHEVNTEV TENDPVSKIF FEQGTYQCLE       420
NCGTVALTII RRGGDLTNTV FVDFRTEDGT ANAGSDYEFT EGTVVFKPGD TQKEIRVGII       480
DDDIFEEDEN FLVHLSNVKV SSEASEDGIL EANHVSTLAC LGSPSTATVT IFDDDHAGIF       540
TFEEPVTHVS ESIGIMEVKV LRTSGARGNV IVPYKTIEGT ARGGGEDFED TCGELEFQND       600
EIVKTISVKV IDDEEYEKNK TFFLEIGEPR LVEMSEKKAL LLNELGGFTI TGKYLFGQPV       660
FRKVHAREHP ILSTVITIAD EYDDKQPLTS KEEEERRIAE MGRPILGEHT KLEVIIEESY       720
EFKSTVDKLI KKTNLALVVG TNSWREQFIE AITVSAGEDD DDDECGEEKL PSCFDYVMHF       780
LTVFWKVLFA FVPPTEYWNG WACFIVSILM IGLLTAFIGD LASHFGCTIG LKDSVTAVVF       840
VALGTSVPDT FASKVAATQD QYADASIGNV TGSNAVNVFL GIGVAWSIAA IYHAANGEQF       900
KVSPGTLAFS VTLFTIFAFI NVGVLLYRRR PEIGGELGGP RTAKLLTSCL FVLLWLLYIF       960
FSSLEAYCHI KGF                                                         973
```

(b) SEQ ID NO: 2

```
MAPLALVGVT LLLAAPPCSG AATPTPSLPP PPANDSDTST GGCQGSYRCQ PGVLLPVWEP        60
DDPSLGDKAA RAVVYFVAMV YMFLGVSIIA DRFMAAIEVI TSKEKEITIT KANGETSVGT       120
VRIWNETVSN LTLMALGSSA PEILLSVIEV CGHNFQAGEL GPGTIVGSAA FNMFVVIAVC       180
IYVIPAGESR KIKHLRVFFV TASWSIFAYW LYLILAVFS PGVVQVWEAL LTLVFFPVCV       240
VFAWMADKRL LFYKYVYKRY RTDPRSGIII GAEGDPPKSI ELDGTFVGAE APGELGGLGP       300
GPAEARELDA SRREVIQILK DLKQKHPDKD LEQLVGIANY YALLHQQKSR AFYRIQATRL       360
MTGAGNVLRR HAADASRRAA PAEGAGEDED DGASRIFFEP SLYHCLENCG SVLLSVTCQG       420
GEGNSTFYVD YRTEDGSAKA GSDYEYSEGT LVFKPGETQK ELRIGIIDDD IFEEDEHFFV       480
RLLNLRVGDA QGMFEPDGGG RPKGRLVAPL LATVTILDDD HAGIFSFQDR LLHVSECMGT       540
VDVRVVRSSG ARGTVRLPYR TVDGTARGGG VHYEDACGEL EFGDDETMKT LQVKIVDDEE       600
YEKKDNFFIE LGQPQWLKRG ISALLLNQGD GDRKLTAEEE EARRIAEMGK PVLGENCRLE       660
VIIEESYDFK NTVDKLIKKT NLALVIGTHS WREQFLEAIT VSAGDEEEEE DGSREERLPS       720
CFDYVMHFLT VFWKVLFACV PPTEYCHGWA CFGVSILVIG LLTALIGDLA SHFGCTVGLK       780
DSVNAVVFVA LGTSIPDTFA SKVAALQDQC ADASIGNVTG SNAVNVFLGL GVAWSVAAVY       840
WAVQGRPFEV RTGTLAFSVT LFTVFAFVGI AVLLYRRRPH IGGELGGPRG PKLATTALFL       900
GLWLLYILFA SLEAYCHIRG F                                                921
```

## (c) SEQ ID NO: 3

```
MAWLRLQPLT SAFLHFGLVT FVLFLNGLRA EAGGSGDVPS TGQNNESCSG SSDCKEGVIL      60
PIWYPENPSL GDKIARVIVY FVALIYMFLG VSIIADRFMA SIEVITSQER EVTIKKPNGE     120
TSTTTIRVWN ETVSNLTLMA LGSSAPEILL SLIEVCGHGF IAGDLGPSTI VGSAAFNMFI     180
IIGICVYVIP DGETRKIKHL RVFFITAAWS IFAYIWLYMI LAVFSPGVVQ VWEGLLTLFF     240
FPVCVLLAWV ADKRLLFYKY MHKKYRTDKH RGIIIETEGD HPKGIEMDGK MMNSHFLDGN     300
LVPLEGKEVD ESRREMIRIL KDLKQKHPEK DLDQLVEMAN YYALSHQQKS RAFYRIQATR     360
MMTGAGNILK KHAAEQAKKA SSMSEVHTDE PEDFISKVFF DPCSYQCLEN CGAVLLTVVR     420
KGGDMSKTMY VDYKTEDGSA NAGADYEFTE GTVVLKPGET QKEFSVGIID DDIFEEDEHF     480
FVRLSNVRIE EEQPEEGMPP AIFNSLPLPR AVLASPCVAT VTILDDDHAG IFTFECDTIH     540
VSESIGVMEV KVLRTSGARG TVIVPFRTVE GTAKGGGEDF EDTYGELEFK NDETVKTIRV     600
KIVDEEEYER QENFFIALGE PKWMERGISA LLLSPDRKLT MEEEEAKRIA EMGKPVLGEH     660
PKLEVIIEES YEFKTTVDKL IKKTNLALVV GTHSWRDQFM EAITVSAAGD EDEDESGEER     720
LPSCFDYVMH FLTVFWKVLF ACVPPTEYCH GWACFAVSIL IIGMLTAIIG DLASHFGCTI     780
GLKDSVTAVV FVAFGTSVPD TFASKAAALQ DVYADASIGN VTGSNAVNVF LGIGLAWSVA     840
AIYWALQGQE FHVSAGTLAF SVTLFTIFAF VCISVLLYRR RPHLGGELGG PRGCKLATTW     900
LFVSLWLLYI LFATLEAYCY IKGF                                          924
```

Figure 2:

|  | C-slow [pF] | Peak current [pA] | Rel. peak current [pA/pF] |
|---|---|---|---|
| Mean | 13.00 | 1.00 | 0.12 |
| SEM | 4.73 | 0.58 | 0.07 |

Figure 3:

CHO-control

Figure 4:

(a)

|  | C-slow [pF] | Peak current [pA] | Rel. peak current [pA/pF] | Transported charge [pC] | Rel. charge [pC/pF] |
|---|---|---|---|---|---|
| Mean | 26.20 | 38.33 | 1.40 | 146.48 | 5.36 |
| SEM | 1.93 | 2.85 | 0.18 | 12.49 | 0.78 |

(b)

| | C-slow [pF] | Peak current [pA] | Rel. peak current [pA/pF] | Transported charge [pC] | Rel. charge [pC/pF] |
|---|---|---|---|---|---|
| Mean | 22.17 | 59.50 | 2.95 | 119.23 | 5.99 |
| SEM | 2.57 | 10.90 | 0.70 | 27.10 | 1.67 |

(c)

| | C-slow [pF] | Peak current [pA] | Rel. peak current [pA/pF] | Transported charge [pC] | Rel. charge [pC/pF] |
|---|---|---|---|---|---|
| Mean | 32.35 | 105.75 | 3.46 | 171.53 | 5.84 |
| SEM | 5.60 | 28.25 | 1.11 | 52.93 | 2.25 |

Figure 5:

(a)

(NCX#1)

(b)

(NCX#2)

(c)

(NCX#3)

Figure 6:

(a)

(b)

Figure 7:

(a) (b)

Figure 8:

Figure 9:

(a) (b)

Figure 10:

(a) (b)

Figure 11:

(a)                       (b)

Figure 12:

(a)                       (b)

Figure 13:

(a)                       (b)

Figure 14:

Figure 15:

(a)

(b)

Figure 16:

Figure 17:

(a)

(b)

Figure 18:

Figure 19:

(a)                                  (b)

Figure 20:

(a)                                  (b)

**European Patent Office**

**EUROPEAN SEARCH REPORT**

Application Number

EP 08 29 0265

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | KIEDROWSKI LECH ET AL: "Differential contribution of plasmalemmal Na/Ca exchange isoforms to sodium-dependent calcium influx and NMDA excitotoxicity in depolarized neurons." JOURNAL OF NEUROCHEMISTRY JUL 2004, vol. 90, no. 1, July 2004 (2004-07), pages 117-128, XP002488542 ISSN: 0022-3042 * page 118, column 1, last paragraph * * page 118, column 2, paragraphs 2,L; figures 1a,1c,2e,2f * * page 126, column 2, last paragraph * ----- | 1-28 | INV. G01N33/58 G01N33/68 |
| X | NAMEKATA ET AL: "Reduction by SEA0400 of myocardial ischemia-induced cytoplasmic and mitochondrial Ca<2+> overload" EUROPEAN JOURNAL OF PHARMACOLOGY, AMSTERDAM, NL, vol. 543, no. 1-3, 14 August 2006 (2006-08-14), pages 108-115, XP005573423 ISSN: 0014-2999 * page 115, column 1, lines 11-16 * * page 111, paragraphs 3.4,3.5; figure 3 * * page 110, column 1, lines 4-8 * ----- -/-- | 1-28 | TECHNICAL FIELDS SEARCHED (IPC) G01N |

The present search report has been drawn up for all claims

| Place of search | Date of completion of the search | Examiner |
|---|---|---|
| The Hague | 16 July 2008 | Vadot-Van Geldre, E |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

**European Patent Office**

**EUROPEAN SEARCH REPORT**

Application Number

EP 08 29 0265

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | KIANG J G ET AL: "Sodium cyanide increases cytosolic free calcium: evidence for activation of the reversed mode of the Na+/Ca2+ exchanger and Ca2+ mobilization from inositol trisphosphate-insensitive pools." TOXICOLOGY AND APPLIED PHARMACOLOGY AUG 1994, vol. 127, no. 2, August 1994 (1994-08), pages 173-181, XP002488543 ISSN: 0041-008X * figures 1,3,7; table 3 * ----- | 1-28 | |
| X | ALTIMIMI HAIDER F ET AL: "Na+-dependent inactivation of the retinal cone/brain Na+/Ca2+-K+ exchanger NCKX2." THE JOURNAL OF BIOLOGICAL CHEMISTRY 9 FEB 2007, vol. 282, no. 6, 9 February 2007 (2007-02-09), pages 3720-3729, XP002488544 ISSN: 0021-9258 * page 3721, column 1, paragraphs BRIDGING,COL,2; figures 1-3 * ----- | 1-28 | TECHNICAL FIELDS SEARCHED (IPC) |
| X | EP 1 526 140 A (YAMANOUCHI PHARMA CO LTD [JP] ASTELLAS PHARMA INC [JP]) 27 April 2005 (2005-04-27) * paragraphs [0002], [0023], [0064], [0065]; example 4 * ----- | 1-28 | |

The present search report has been drawn up for all claims

| Place of search | Date of completion of the search | Examiner |
|---|---|---|
| The Hague | 16 July 2008 | Vadot-Van Geldre, E |

CATEGORY OF CITED DOCUMENTS

X : particularly relevant if taken alone
Y : particularly relevant if combined with another
    document of the same category
A : technological background
O : non-written disclosure
P : intermediate document

T : theory or principle underlying the invention
E : earlier patent document, but published on, or
    after the filing date
D : document cited in the application
L : document cited for other reasons

    ........................................................................................................
& : member of the same patent family, corresponding
    document

EPO FORM 1503 03.82 (P04C01)

## ANNEX TO THE EUROPEAN SEARCH REPORT
## ON EUROPEAN PATENT APPLICATION NO.

EP 08 29 0265

This annex lists the patent family members relating to the patent documents cited in the above-mentioned European search report.
The members are as contained in the European Patent Office EDP file on
The European Patent Office is in no way liable for these particulars which are merely given for the purpose of information.

16-07-2008

| Patent document cited in search report | | Publication date | Patent family member(s) | | Publication date |
|---|---|---|---|---|---|
| EP 1526140 | A | 27-04-2005 | AU | 2003252754 A1 | 23-02-2004 |
| | | | CA | 2480427 A1 | 12-02-2004 |
| | | | WO | 2004013173 A1 | 12-02-2004 |
| | | | US | 2005119173 A1 | 02-06-2005 |

EPO FORM P0459

For more details about this annex : see Official Journal of the European Patent Office, No. 12/82

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- EP 1031556 A

- US 5714666 A

**Non-patent literature cited in the description**

- **Flesch et al.** *Circulation,* 1996
- **Komuro ; Ohtsuka.** *Journal of Pharmacological. Sciences,* 2004
- **Hobai, JA ; O'Rourke,B.** *Expert Opin. Investig. Drugs,* 2004, vol. 13, 653-664
- **Iwamoto, T. et al.** *J. Biol. Chem.,* 2004, vol. 279, 7544-7553
- **K. Acsai.** ESC Congress 2004. *Munich on Poster Nr. 2886,* 2004
- **C. Lee et al.** *The journal of pharmacology and experimental therapeutics,* 2004, vol. 311, 748-757
- **Tong Mook Kang ; Donald W. Hilgemann.** *Nature,* 05 February 2004, vol. 427
- **T. Kuramochi et al.** *Bioorganic & Medicinal Chemistry,* 2004, vol. 12, 5039-5056
- **Nicoll, DA. et al.** *Science,* 1990, vol. 250 (4980), 562-5
- **Komuro, I. et al.** *Proc. Natl. Acad. Sci. U.S.A.,* 1992, vol. 89 (10), 4769-4773
- **Kofuji, P. et al.** Am. J. Physiol. *Cell Physiol. 32,* 1992, vol. 263, C1241-C1249
- **Li, Z. et al.** *J. Biol. Chem.,* 1994, vol. 269 (26), 17434-9
- **Nicoll, DA.** *J. Biol. Chem.,* 1996, vol. 271 (40), 24914-21
- **Gabellini, N.** *Gene,* 2002, vol. 298, 1-7
- Current protocols in cell biology. John Wiley & Sons Inc
- **Créton et al.** *Microscopy Research and Technique,* 1999, vol. 46, 390-397
- **Brini et al.** *J. Biol. Chem.,* 1995, vol. 270, 9896-9903
- **Knight ; Knight.** *Meth. Cell. Biol.,* 1995, vol. 49, 201-216
- Current protocols in molecular biology. John Wiley & Sons Inc
- **Kiedrowski et al.** *Journal of Neurochemistry,* 2004